(19) Europäisches Patentamt
European Patent Office
Office européen des brevets

(11) Publication number: **0 170 430**
**A1**

(12) # EUROPEAN PATENT APPLICATION

(21) Application number: 85304762.9

(22) Date of filing: 04.07.85

(51) Int. Cl.[4]: C 07 D 265/32
A 61 K 31/535

(30) Priority: 05.07.84 GB 8417170

(43) Date of publication of application: 05.02.86 Bulletin 86/6

(84) Designated Contracting States: AT BE CH DE FR GB IT LI LU NL SE

(71) Applicant: THE WELLCOME FOUNDATION LIMITED
183-193 Euston Road
London NW1 2BP(GB)

(72) Inventor: Mehta, Nariman Bomanshaw
4207 Union Street
Raleigh North Carolina(US)

(72) Inventor: Smyser, Thomas Edgar
6410 Arrington Road
Raleigh North Carolina(US)

(54) **Heterocyclic pharmaceutical compounds, preparation and use.**

(57) Morpholinol compounds of formula (I)

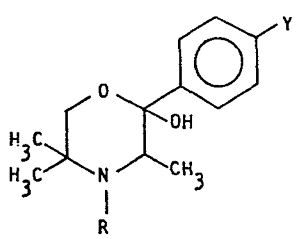

I

wherein Y is hydrogen or fluorine and R is hydrogen or alkyl ($C_{1-4}$), and salts thereof.

These compounds are of value in both human and veterinary medicine, for example in the prophylaxis or treatment of a depressed state in mammals including human beings.

The invention is also directed to methods for the preparation of the compounds, to pharmaceutical formulations containing them and the preparation thereof, and to the use of the compounds in medicine.

EP 0 170 430 A1

# Heterocyclic Pharmaceutical Compounds, Preparation and Use

The present invention relates to novel morpholinols useful in medicine, to processes for preparing them, to pharmaceutical formulations containing them and their preparation, to the use of the compounds in medicine and to novel chemical intermediates therefor and the preparation thereof.

It has been found that the novel morpholinol compounds represented by formula (I)

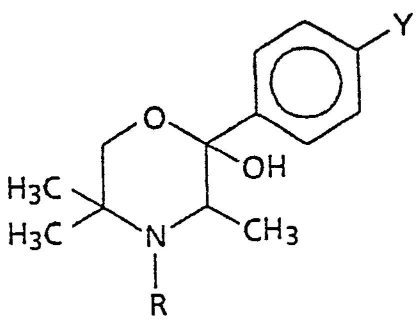

I

wherein Y is hydrogen or fluorine and R is hydrogen or alkyl ($C_{1-4}$), and salts thereof, have antidepressant activity as demonstrated by widely accepted techniques used in the art of pharmacology for determining antidepressant activity, for example, the tetrabenazine-induced sedation test in rodents. Advantageously the compounds of this invention do not produce any significant degree of locomotor stimulation and are essentially free of proconvulsant activity even at doses near to or in excess of the $LD_{50}$.

Structural formula (I) should be understood to extend to and embrace all geometric and optical isomers.

As preferred within formula (I) may be mentioned :

(i) compounds wherein R is hydrogen or alkyl ($C_{2-4}$)

(ii) compounds having the cis configuration

(iii) compounds wherein R is hydrogen

(iv) cis-3,5,5-trimethyl-2-phenyl-2-morpholinol (compound IA)

(v) cis-2-(4-fluorophenyl)-3,5,5-trimethyl-2-morpholinol (compound IB)

and salts thereof.

The compounds of formula (I) may be synthesized by the methods known in the art for the preparation of compounds having this type of structure.

(1) Thus as one possibility a compound of formula (I) can be made by reacting a compound of formula (II) with a compound of formula (III) [wherein Y and R are as defined for formula (I) and L is a suitable leaving atom or group such as halo (for example, bromo) or arylsulfonyloxy (for example, p-toluenesulfonyloxy) ] in a suitable solvent such as acetonitrile, ethanol, methanol or dichloromethane.

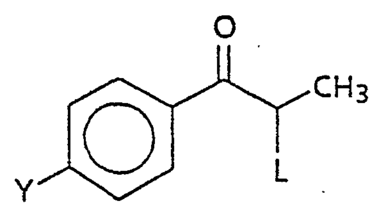

II

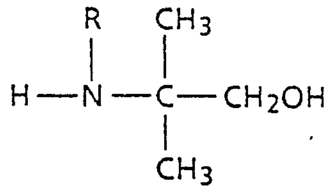

III

( 2) Compounds of formula (I) wherein R is alkyl can also be made by either alkylating, or acylating and then selectively reducing, the corresponding compound of formula (I) wherein R is hydrogen, using methods well known in the art of organic chemistry. For example, alkylation with methyliodide gives the compounds of formula (I) where R is methyl, acylation with acetyl chloride followed by reduction with diborane in tetrahydrofuran gives the compounds where R is ethyl.

The compounds of formula (I) and their pharmaceutically acceptable salts may be used in the prophylaxis or treatment of a depressed state in mammals including human beings, the treatment comprising the administration of an antidepressant effective, non-toxic amount (dose), preferably in a unit dosage form, of a compound of formula (I) or a pharmaceutically acceptable salt thereof. The compounds and salts may also be used to treat human beings for tardive dyskinesia (TD), and minimal brain dysfunction (MBD).

The preferred dosage for parenteral (including subcutaneous, intramuscular and intravenous) administration of a compound of formula (I) or salt thereof (estimated as the base) is in the range 5 mg/kg to 50 mg/kg of mammal body weight, the most preferred dosage being 15 mg/kg to 35 mg/kg of mammal body weight.

For the oral or rectal mode of administration, the preferred dosage of a compound of formula (I) or salt thereof (estimated as the base) is in the range 10 mg/kg to 100 mg/kg of mammal body weight while the most preferred dosage is 30 mg/kg to 70 mg/kg of mammal body weight.

For the treatment of human beings, the preferred unit dosage of a compound of formula (I) or salt thereof (estimated as the base) for oral or rectal administration is in the range 15 mg to 500 mg with the more preferred unit dosage being in the range 100 mg to 300 mg, and the most preferred unit dosage being in the range 125 mg to 250 mg. For parenteral treatment the preferred unit dosage is 4 mg to 200 mg with the more preferred unit dosage being in the range 50 mg to 175 mg, and the most preferred unit dosage being in the range of 100 mg to 150 mg.

All the above doses are given in terms of the weight of the base but the compound of formula (I) is preferably administered in the form of a pharmaceutically acceptable salt thereof.

A compound of formula (I) or salt thereof is preferably administered four times daily although this may vary according to the patient (mammal) being treated, and the exercise of the physician's discretion.

While it is possible for the active compound, i.e., compound of formula (I) or pharmaceutically acceptable salt thereof, to be administered alone as the raw chemical, it is preferable to present it as a pharmaceutical formulation comprising a compound of formula (I) (or a pharmaceutically acceptable salt thereof) together with a pharmaceutically acceptable carrier therefor.

The carrier should be acceptable in the sense of being compatible with the other ingredients of the formulation and not deleterious to the recipient thereof.

Conveniently the active compound comprises from 5 to 95% by weight of the formulation.

The formulations include those suitable for oral, rectal or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product.

Formulations of the present invention suitable for oral administration may be presented as discrete units such as capsules, cachets, tablets or lozenges, each containing a predetermined amount of the active compound; as a powder or granules including microencapsulated or time-release forms; or as a suspension or solution in an aqueous liquid or non-aqueous liquid such as a syrup, an elixir, an emulsion or a draught.

A tablet may be made by compression or molding, optionally with one or more accessory ingredients. Compressed tablets may be prepared by compressing in

a suitable machine, the active compound being in a free-flowing form such as a powder or granules optionally mixed with a binder, disintegrant, lubricant, inert diluent, surface active agent or dispersing agent. Molded tablets comprised of a mixture of the powdered active compound with any suitable carrier may be made by molding in a suitable machine.

Formulations suitable for rectal administration may be presented as a suppository with a conventional carrier such as cocoa butter, hydrogenated fats or hydrogenated fatty carboxylic acids.

Formulations suitable for parenteral administration conveniently comprise a sterile aqueous preparation of the active compound which is preferably isotonic with the blood of the intended recipient. The formulations may be presented in unit-dose or multi-dose containers, for example sealed ampoules and vials, and may be stored in a freeze-dried state requiring only the addition of the sterile liquid carrier, for example water, just prior to use.

In addition to the aforementioned ingredients, the formulations of this invention may further include one or more accessory ingredient(s) selected as appropriate from diluents, buffers, flavouring agents, binders, disintegrants, surface active agents, thickeners, lubricants, preservatives (including antioxidants) and the like.

When used in medicine, the salts of a compound of formula (I) should be pharmaceutically acceptable, but non pharmaceutically acceptable salts may conveniently be used to prepare the corresponding free base or pharmaceutically acceptable salts thereof and are included within the scope of this invention.

Such pharmaceutically acceptable salts include, but are not limited to, those prepared from the following acids: hydrochloric, hydrobromic, sulfuric, nitric, phosphoric, salicylic, p-toluenesulfonic, tartaric, citric, methanesulfonic, maleic, formic, malonic, succinic, isethionic, lactobionic, naphthalene-2-sulfonic, sulfamic, ethanesulfonic and benzenesulfonic.

The following Examples are provided by way of illustration of the present invention and should in no way be construed as a limitation thereof. All temperatures indicated are in degrees Celsius.

Example 1: *cis*-3,5,5-Trimethyl-2-phenyl-2-morpholinol hydrochloride

To a solution of 2-amino-2-methyl-1-propanol (7.5 g) in 80 ml acetonitrile was added a solution of α-bromopropiophenone (5.6 g) in 50 ml acetonitrile. The resulting solution was stirred overnight at room temperature. The reaction mixture was concentrated and partitioned between ether and water. The ether phase was extracted with 10% hydrochloric acid. The aqueous acid phase was basified with sodium hydroxide to yield *cis*-3,5,5-trimethyl-2-phenyl-2-morpholinol. The free base was dissolved in ether and treated with ethereal hydrochloric acid to give the hydrochloride salt, m.p. 199-200°C (after recrystallization from ethanol/ether). Elemental Analysis: Calcd. for $C_{13}H_{20}ClNO_2$ (m.w. 257.76). C, 60.57%; H, 7.82%; N, 5.44%. Found: C, 60.63%; H, 7.86%; N, 5.42%. The IR spectrum did not show any characteristic absorption in the carbonyl region. The UV spectrum showed the absence of a significant absorption in the carbonyl region above 220 nm.

The H and $^{13}C$ NMR spectra were consistent with the assigned structure. Esylate (ethanesulphonate) salt m.p. 189-190°C dec. Besylate (benzenesulphonate) salt m.p. 159-161°C dec.

Example 2: cis-2-(4-Fluorophenyl)-3,5,5-trimethyl-2-morpholinol hydrochloride

To a solution of 2-bromo-4'-fluoropropiophenone (40.0 g) in 200 ml acetonitrile was added a solution of 2-amino-2-methyl-1-propanol (46.3 g) in 200 ml acetonitrile. The resulting solution was stirred at room temperature for eight days. The reaction mixture was concentrated and partitioned between ether and water. The ether phase was extracted with 10% hydrochloric acid. The aqueous acid phase was basified with sodium hydroxide to yield cis-2-(4-fluorophenyl)-3,5,5-trimethyl-2-morpholinol. The free base was dissolved in ether and treated with ethereal hydrochloric acid to give the hydrochloride salt, m.p. 211-212°C (after recrystallization from ethanol/ether). Elemental Analysis: Calcd. for $C_{13}H_{19}ClFNO_2$ (mw 275.75). C, 56.62%, H, 6.95%; N, 5.08%. Found: C, 56.75%; H, 7.02%; N, 5.0%.

Example 3: cis-3,4,5,5-Tetramethyl-2-phenyl-2-morpholinol hydrochloride

To a solution of cis-3,5,5-trimethyl-2-phenyl-2-morpholinol (2.0 g) in 60 ml acetonitrile was added iodomethane (6.4 g). The resulting solution was warmed slightly overnight in a pressure bottle. The reaction mixture was concentrated, dissolved in water and basified with sodium hydroxide to yield cis-3,4,5,5-tetramethyl-2-phenyl-2-morpholinol. The free base was dissolved in ether and treated with ethereal hydrochloric acid to give the hydrochloride salt,

m.p. 195-197°C (after recrystallization from ethanol/ether). Elemental Analysis: Calcd. for $C_{14}H_{22}ClNO_2$ (m.w. 271.79). C, 61.86%; H, 8.16%; N, 5.15%. Found: C, 61.77%; H, 8.18%; N, 5.11%. The NMR spectrum was consistent with the structure.

Example 4 : Antitetrabenazine Test

Prevention of tetrabenazine induced sedation was measured using a modification of the method of Vernier, et al., First Hahnemann Symposium on Psychosomatic Medicine, ed. Nodim and Moyer, pub. Lea and Febiger, Philadelphia, 1962.

Mice, three groups of 12 CD1 males each, were injected intraperitoneally (ip) with a solution of compound (IA) or compound (IB) or with a saline solution alone. Thirty minutes later each of the mice was injected (ip, 35 mg/kg) with a solution containing tetrabenazine hydrochloride. Thirty minutes after the injection of tetrabenazine each mouse was examined for its level of exploratory behavior which was scored on a modification of the arbitrary scale defined by Vernier, et. al. The results reported in Table I as the $ED_{50}$ values are the amounts of the test compounds required to reverse the tetrabenazine effects in 50 percent of the animals tested.

Table I

Effective Antitetrabenazine

Activity in the Mouse

| Compound | $ED_{50}$ (mg/kg i.p.) |
|---|---|
| IA (Ex. 1 HCl) | 18 |
| IB (Ex. 2 HCl) | 14 |

Example 5: Formulations

A. Tablet

| Ingredient | Amount per Tablet |
|---|---|
| A compound of formula (I) (as the base) | 150 mg |
| Lactose | 85 mg |
| Cornstarch | 50 mg |
| Micronized Silica Gel | 10 mg |
| Polyvinylpyrrolidone | 5 mg |

The lactose, cornstarch and compound of formula (I) are mixed together and granulated with a binder (polyvinylpyrrolidone in an alcoholic solution), to form granules. The granules are passed through a 16-20 mesh screen, then air dried, lubricated with micronized silica gel and compressed into tablets. A film coat may then be applied if desired.

B. Capsule

| Ingredient | Amount per Capsule |
|---|---|
| A compound of formula (I) (as the base) | 150 mg |
| Lactose | 125 mg |
| Cornstarch | 125 mg |

The above ingredients are mixed and filled into a two piece hard gelatin capsule.

C. Parenteral Solution

| Ingredient | Amount per Ampoule |
|---|---|
| A compound of formula (I) as a pharmaceutically acceptable salt | 125 mg (based on free base) |
| Sterile Water for Injections, q.s. to | 1.0 mL |

A pharmaceutically acceptable salt of a compound of formula (I) is dissolved in sterile water under sterile conditions to make 1.0 mL. Such a solution may be packaged in a sealed sterile ampoule to provide a unit dose or in a sterile vial for multiple doses. If the formulation is to be packed in a multi-dose container the addition of a bacteriostat such as 0.2 to 0.5% w/v of phenol is desirable.

D. Suppository

150 Mg of the hydrochloride salt of a compound formula (I) is mixed with 250 mg of softened or melted cocoa butter, and a suppository is formed by chilling and shaping in a mold.

Example 6 Toxicity

The acute $LD_{50}$ of both compound (IA) and compound (IB) is approximately 160 mg/kg, i.p., in the mouse calculated as the hydrochloride salt.

0170430

What we claim is:

1. Morpholinol compounds of formula (I)

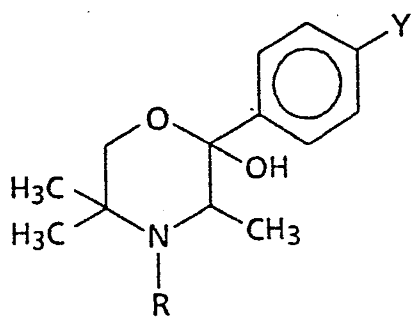

I

wherein

Y is hydrogen or fluorine and

R is hydrogen or alkyl ($C_{1-4}$),

and salts thereof.

2. Compounds according to claim 1 wherein R is hydrogen or alkyl ($C_{2-4}$), and salts thereof.

3. Compounds according to either of claims 1 and 2 having the cis configuration, and salts thereof.

4. Compounds according to any of claims 1 to 3 wherein R is hydrogen, and salts thereof.

5. cis - 3,5,5 - Trimethyl-2-phenyl-2-morpholinol and salts thereof.

6. cis-2-(4-Fluorophenyl)-3,5,5-trimethyl-2- morpholinol and salts thereof.

7. A salt of a compound according to any of claims 1 to 6.

8. A pharmaceutically acceptable salt of a compound according to any of claims 1 to 6.

9. The hydrochloride salt of a compound according to any of claims 1 to 6.

10. A compound according to any of claims 1 to 6, or a pharmaceutically acceptable salt thereof, for use in the medical treatment of a mammal.

11. A compound according to any of claims 1 to 6, or a pharmaceutically acceptable salt thereof, for use in the prophylaxis or treatment of a depressed state in a mammal.

12. The use of a compound according to any of claims 1 to 6, or a pharmaceutically acceptable salt thereof, for the manufacture of a medicament for the prophylaxis or treatment of a depressed state in a mammal.

13. A pharmaceutical formulation comprising a compound according to any of claims 1 to 6, or a pharmaceutically acceptable salt thereof, together with an acceptable carrier therefor.

14. A method for the preparation of a formulation according to claim 13 comprising admixture of the ingredients thereof.

MJC/OLM/5th June 1985

15. A method for the preparation of a compound according to any of claims 1 to 6, or a salt thereof, comprising

(a) reacting a compound of formula (II) with a compound of formula (III)

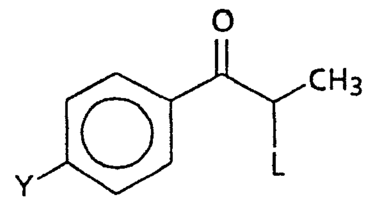

II

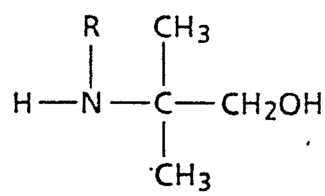

III

wherein Y and R are as defined in formula (I) and L is a leaving atom or group; or

(b) when R is alkyl, alkylating the corresponding compound of formula (I) wherein R is hydrogen; or

(c) when R is alkyl, acylating and then selectively reducing the corresponding compound of formula (I) wherein R is hydrogen;

followed as appropriate by conversion of the product into the free base or a salt thereof.

16. A compound according to any of claims 1 to 6, or a salt thereof, when prepared by a method according to claim 15.

European Patent Office

# EUROPEAN SEARCH REPORT

0170430

Application number

EP 85 30 4762

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | US-A-3 117 967 (E.L.ANDERSON)<br>* The whole document *<br>--- | 1,13,<br>15 | C 07 D 265/32<br>A 61 K 31/535 |
| P,A | EP-A-0 139 590 (LAB. L.LAFON)<br>* Claims *<br>----- | 1,13,<br>15 | |
| | | | TECHNICAL FIELDS SEARCHED (Int. Cl 4) |
| | | | C 07 D 265/00<br>A 61 K 31/00 |

The present search report has been drawn up for all claims

| Place of search | Date of completion of the search | Examiner |
|---|---|---|
| THE HAGUE | 01-10-1985 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document